(19) Europäisches Patentamt
European Patent Office
Office européen des brevets

(11) **EP 4 527 374 A1**

(12) **EUROPEAN PATENT APPLICATION**
published in accordance with Art. 153(4) EPC

(43) Date of publication:
26.03.2025 Bulletin 2025/13

(21) Application number: 23807713.5

(22) Date of filing: 19.05.2023

(51) International Patent Classification (IPC):
A61K 9/08 (2006.01)    A61K 31/7088 (2006.01)
A61K 45/00 (2006.01)    A61K 47/02 (2006.01)
A61K 47/26 (2006.01)    A61K 48/00 (2006.01)
A61M 5/303 (2006.01)    A61P 43/00 (2006.01)

(52) Cooperative Patent Classification (CPC):
A61K 9/08; A61K 31/7088; A61K 45/00;
A61K 47/02; A61K 47/26; A61K 48/00; A61M 5/30;
A61P 43/00

(86) International application number:
PCT/JP2023/018700

(87) International publication number:
WO 2023/224112 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.05.2022 JP 2022082953

(71) Applicant: Daicel Corporation
Osaka-shi, Osaka 530-0011 (JP)

(72) Inventor: SAKAGUCHI, Naoki
Tokyo 108-8230 (JP)

(74) Representative: Grünecker Patent- und
Rechtsanwälte
PartG mbB
Leopoldstraße 4
80802 München (DE)

(54) **LIQUID PHARMACEUTICAL COMPOSITION**

(57) An object of the present disclosure is to provide at least the following. Specifically, provided is a technique in which high physiological activity is achieved when a liquid pharmaceutical composition containing a biofunctional substance that exhibits the physiological activity in an injection target is injected into the injection target. The above-described problem is solved by a liquid pharmaceutical composition containing a biofunctional substance and having an osmotic pressure of more than 400 mOsmol/kg and 1000 mOsmol/kg or less. The liquid pharmaceutical composition is injected into an injection target by an injector configured to inject the liquid pharmaceutical composition into the injection target without using an injection needle.

| | | | | | | | | |
|---|---|---|---|---|---|---|---|---|
| TEST EXAMPLE 1 | | | | | | | | |
| COMPARATIVE TEST EXAMPLE 1 | | | | | | | | |
| NO TREATMENT | 57.00 (TE) | 198.00 | 346.00 | 624.00 | 899.00 | 1154.00 | 1424.00 |

MEASURED OSMOTIC PRESSURE (mOsmol/kg)

FIG. 2

EP 4 527 374 A1

**Description**

TECHNICAL FIELD

The present disclosure relates to a liquid pharmaceutical composition.

BACKGROUND ART

In recent years, in regard to medical devices for administering a medicinal solution, interest in needleless injectors has been increasing from the viewpoint of a countermeasure against needle phobia, pursuit of usability, reduction of infectious wastes, and the like. In general, a needleless injector is known as an injector that uses compressed gas or a spring force as a driving force to push out a medicinal solution from a nozzle tip, thereby administering the medicinal solution into a living body (Non-Patent Literature 1). Further, a needleless injector that uses combustion energy of an ignition agent as ejection energy has been developed (Patent Document 1).

The needleless injector that uses combustion energy of an ignition agent as ejection energy is capable of delivering a medicinal solution to the nucleus or cytosol of a cell because the medicinal solution is instantaneously pushed out and administered. The usefulness of the needleless injector has been attracting attention also from the viewpoint of a drug delivery system (DDS), and application of the needleless injector to various drugs using a low molecular weight compound, a peptide, a protein, an antibody, or the like having an anticancer action has been studied. In particular, in the fields of vaccines and immunization, it has been reported that the needleless injector enables an increase in antibody titer or the induction of cellular immunity (Non-Patent Literature 2).

Also in the field of drug discovery, it is well known that modalities of nucleic acid pharmaceuticals such as DNA and RNA generally require delivery of an active ingredient to the cytosol of a cell. For this reason, the use of electroporation, viral vectors, lipid nanoparticles (LNPs), and the like has been studied. Further, the needleless injector that uses combustion energy of an ignition agent as ejection energy has also been studied as means for delivering DNA or RNA to the cytosol and achieving high drug efficacy (Non-Patent Literature 2).

Meanwhile, viral vectors and lipid nanoparticles have a problem that they can induce side effects such as anaphylaxis in a subject to which they are administered, and there is a need for a technique that does not use them. Electroporation also has clinical problems such as the occurrence of local toxicity and the use of complex machinery. Further, the needleless injector that uses combustion energy of an ignition agent as ejection energy has been improved for increasing the amount of a nucleic acid pharmaceutical delivered to the cytosol, gene expression efficiency, and the expression efficiency of drug efficacy.

CITATION LIST

PATENT DOCUMENT

Patent Document 1: JP 2012-61269 A

NON-PATENT LITERATURE

Non-Patent Literature 1: Clin. Cosmet. Investig. Dermatol., 2018 May 1; 11: 231 to 238
Non-Patent Literature 2: AAPS PharmSciTech., 2019 Dec 9; 21(1): 19

SUMMARY OF INVENTION

TECHNICAL PROBLEM

An object of the present disclosure is to provide at least the following. That is, there is provided a technique capable of achieving high physiological activity when a liquid pharmaceutical composition containing a biofunctional substance exhibiting physiological activity in an injection target is injected into the injection target.

SOLUTION TO PROBLEM

The present inventor has found that in a case where a liquid pharmaceutical composition is injected into an injection target using an injector configured to inject the liquid pharmaceutical composition into the injection target without using an injection needle, the above-described problem can be solved when an osmotic pressure of the liquid pharmaceutical composition is set in a predetermined range.

One aspect of the present disclosure is a liquid pharmaceutical composition containing:

a biofunctional substance, wherein
the liquid pharmaceutical composition has an osmotic pressure of more than 400 mOsmol/kg and 1000 mOsmol/kg or less, and
the liquid pharmaceutical composition is injected into an injection target by an injector configured to inject the liquid pharmaceutical composition into the injection target without using an injection needle.

In a preferred embodiment, the liquid pharmaceutical composition contains a pharmacologically acceptable agent.

In another preferred embodiment of the liquid pharmaceutical composition, the pharmacologically acceptable agent is an ionic agent.

In another preferred embodiment of the liquid pharmaceutical composition, the ionic agent is a buffer solution containing phosphoric acid.

In another preferred embodiment of the liquid pharmaceutical composition, the buffer solution containing phosphoric acid is phosphate buffered saline.

In another preferred embodiment of the liquid pharmaceutical composition, the ionic agent is sodium chloride.

In another preferred embodiment of the liquid pharmaceutical composition, the pharmacologically acceptable agent is a nonionic agent.

In another preferred embodiment of the liquid pharmaceutical composition, the nonionic agent is glucose.

In another preferred embodiment, injection of the liquid pharmaceutical composition into the injection target using the injector configured to inject the liquid pharmaceutical composition into the injection target without using an injection needle is injection into the injection target by jet injection.

In another preferred embodiment of the liquid pharmaceutical composition, the biofunctional substance is a nucleic acid.

Another aspect of the present disclosure is
an injector configured to inject a liquid pharmaceutical composition into an injection target without using an injection needle, the injector including:

a storage section containing the liquid pharmaceutical composition;
a nozzle section communicating with the storage section, the nozzle section having an ejection port for ejecting the liquid pharmaceutical composition toward the injection target; and
a pressurization section for pressurizing the liquid pharmaceutical composition contained in the storage section during an operation, thereby ejecting the liquid pharmaceutical composition from the ejection port toward the injection target, wherein
the liquid pharmaceutical composition contains a biofunctional substance, and has an osmotic pressure of more than 400 mOsmol/kg and 1000 mOsmol/kg or less.

Another aspect of the present disclosure is
a method for injecting a liquid pharmaceutical composition into an injection target, the method including injecting a liquid pharmaceutical composition into an injection target by an injector configured to inject the liquid pharmaceutical composition into the injection target without using an injection needle, the liquid pharmaceutical composition containing a biofunctional substance and having an osmotic pressure of more than 400 mOsmol/kg and 1000 mOsmol/kg or less.

ADVANTAGEOUS EFFECTS OF INVENTION

The present disclosure can exhibit at least the following effect. Specifically, there can be exhibited an effect of providing a technique capable of achieving high physiological activity when a liquid pharmaceutical composition containing a biofunctional substance exhibiting physiological activity in an injection target is injected into the injection target.

BRIEF DESCRIPTION OF DRAWINGS

FIG. 1 is a diagram illustrating a schematic configuration of an injector according to an embodiment of the present disclosure.

FIG. 2 shows images (photographs substituted for drawings) illustrating the results of Test Example 1 and Comparative Test Example 1 according to an embodiment of the present disclosure.

FIG. 3 shows images (photographs substituted for drawings) illustrating the results of Test Example 2 and Comparative Test Example 2 according to an embodiment of the present disclosure.

FIG. 4 shows images (photographs substituted for drawings) illustrating the results of Test Example 3 and Comparative Test Example 3 according to an embodiment of the present disclosure.

FIG. 5 shows images (photographs substituted for drawings) illustrating the results of Test Example 4 according to an embodiment of the present disclosure.

FIG. 6 shows images (photographs substituted for drawings) illustrating the results of Test Example 5 and Comparative Test Example 5 according to an embodiment of the present disclosure.

DESCRIPTION OF EMBODIMENTS

The configurations, combinations thereof, and the like in the respective embodiments are examples, and various additions, omissions, substitutions, and other changes of the configurations may be made as appropriate without departing from the spirit of the present disclosure. The present disclosure is not limited by the embodiments and is limited only by the claims. Each embodiment disclosed in the present specification can be combined with any other feature disclosed herein.

One embodiment of the present disclosure is a liquid pharmaceutical composition containing:

a biofunctional substance, wherein
the liquid pharmaceutical composition has an osmotic pressure of more than 400 mOsmol/kg and 1000 mOsmol/kg or less, and
the liquid pharmaceutical composition is injected into an injection target using an injector configured to inject the liquid pharmaceutical composition into the injection target without using an injection needle.

(Biofunctional Substance)

The biofunctional substance contained in the liquid pharmaceutical composition according to the present embodiment is a substance exhibiting physiological activity in an injection target. The biofunctional substance contained in the liquid pharmaceutical composition may be one type or a plurality of types. The biofunctional substance may be a natural product or an artificially synthesized product.

Examples of the biofunctional substance include a nucleic acid, a peptide, and a protein.

When the biofunctional substance is a nucleic acid, the nucleic acid may be DNA or RNA. The nucleic acid may be a nucleic acid including a portion encoding a protein, or a nucleic acid not including a portion encoding a protein (non-coding nucleic acid).

Examples of a case where the nucleic acid is a nucleic acid including a portion encoding a protein are as follows.

For example, when the biofunctional substance is DNA containing a gene, the biofunctional substance exhibits physiological activity of serving as a template in synthesis (transcription) of mRNA for protein production, and the physiological activity can be quantitatively evaluated by the amount of mRNA or protein produced.

When the biofunctional substance is mRNA, the biofunctional substance exhibits physiological activity of serving as a template in protein synthesis (translation), and the physiological activity can be quantitatively evaluated by the amount of protein produced.

The physiological activity may be evaluated based on a change in an injection target caused due to an increase or decrease in the produced mRNA or protein. For example, in a case where the injection target is an injection target in which cellular immunity can be induced (for example, an individual (living body)), the nucleic acid is a nucleic acid encoding a protein that induces cellular immunity, and an increase in the protein that induces cellular immunity causes a change in which cellular immunity is induced in the injection target, the nucleic acid exhibits physiological activity of inducing cellular immunity, and the physiological activity can be quantitatively evaluated by the amount of cellular immunity induced in the injection target. The physiological activity may also be evaluated by the amount of cellular immunity induced in the injection target after restimulation (resensitization) with the protein that induces cellular immunity.

Examples of a case where the nucleic acid is a nucleic acid not including a portion encoding a protein are as follows.

For example, in a case where the injection target is an individual (living body) to which a vaccine can be administered, and the nucleic acid is CpG motif-containing DNA (CpG DNA) or polyinosine-polycytidylic acid (PolyI:C), the nucleic acid exhibits physiological activity as an adjuvant for enhancing a vaccine effect, and the physiological activity can be quantitatively evaluated by an antitumor effect (the effect can be evaluated by, for example, a decrease in tumor size), the degree of maturation of immune cells, or the like.

When the nucleic acid is small interfering RNA (siRNA), the nucleic acid exhibits physiological activity of degrading mRNA and suppressing expression of a target gene in a sequence-specific manner, and the physiological activity can be quantitatively evaluated by the amount of mRNA or protein produced based on the target gene.

When the nucleic acid is antisense DNA or antisense RNA, the nucleic acid exhibits physiological activity such as inhibition of splicing or inhibition of translation by hybridizing with RNA of a target gene, and the physiological activity can be quantitatively evaluated by the amount of mRNA or protein produced based on the target gene.

The biofunctional substance according to the present embodiment may be in a free form or in a form fixed to a carrier such as nanoparticles, or in a modified form, and is not particularly limited, including a solvent, as long as when the biofunctional substance is injected into the injection target, the biofunctional substance exhibits physiological activity in the injection target, is stably present in the injection target, and does not have an adverse effect such as destruction of the injection target.

Examples described below include an example in which free plasmid DNA containing a green fluorescence protein (GFP) gene is used as the biofunctional substance and the GFP gene is used as a reporter gene, and an example in which mRNA encoding GFP as a reporter protein is used.

In a case where the DNA contains a gene, it may be designed such that the gene is contained in an expression cassette or an expression vector. Furthermore, for example, the gene may be placed under control of a promoter suitable for the injection target and the injection site into which the DNA is to be injected. That is, known genetic engineering techniques can be used in any of the embodiments. For example, in Examples described below, CMV-DASHER-GFP (available from ATUM), which is a vector containing a GFP gene, is used as an expression vector. The plasmid vector is known and available to those skilled in the art. Subcloning of the expression vector and a recombinant vector can be performed according to a known method.

When the biofunctional substance is a peptide or a protein, examples of the peptide or the protein include an antigen (i.e., a substance producing an antibody against the peptide or the protein), an antibody, a peptide vaccine, a protein vaccine, a peptide hormone, a protein hormone, a growth factor, a cytokine, a blood coagulation factor, serum albumin, a digestive enzyme, an anti-inflammatory peptide, an anti-inflammatory protein, and an antibiotic.

In the present embodiment, the phrase "capable of achieving high physiological activity when a liquid pharmaceutical composition containing a biofunctional substance exhibiting the physiological activity in an injection target is injected into the injection target" means that in a case where the physiological activity exhibited by the biofunctional substance is evaluated using a quantitative index,

the physiological activity (the physiological activity may be, for example, an amount indicating the physiological activity) exhibited by a biofunctional substance when a liquid pharmaceutical composition containing the biofunctional substance and having an osmotic pressure outside a predetermined range described below is injected into an injection target using an injector configured to inject the liquid pharmaceutical composition into the injection target without using an injection needle is surpassed by

the physiological activity (the physiological activity may be, for example, an amount indicating the physiological activity) exhibited by a biofunctional substance when a liquid pharmaceutical composition containing the biofunctional substance and having an osmotic pressure within the predetermined range described below is injected in the same manner as described above.

That is, for example,

in a case where the physiological activity exhibited by a biofunctional substance when a liquid pharmaceutical composition containing the biofunctional substance and having an osmotic pressure of 400 mOsmol/kg or less or more than 1000 mOsmol/kg is injected into an injection target using an injector configured to inject the liquid pharmaceutical composition into the injection target without using an injection needle (this is referred to as embodiment B) is defined as "activity B", and

the physiological activity exhibited by a biofunctional substance when a liquid pharmaceutical composition is injected into an injection target in the same manner as described above under the same conditions as in the embodiment B except that the liquid pharmaceutical composition contains the biofunctional substance and has an osmotic pressure of more than 400 mOsmol/kg and 1000 mOsmol/kg or less (this is referred to as embodiment A) is defined as "activity A",

$$\text{activity B} < \text{activity A}$$

is satisfied.

Alternatively, in the present embodiment, the phrase "capable of achieving high physiological activity when a liquid pharmaceutical composition containing a biofunctional substance exhibiting the physiological activity in an injection target is injected into the injection target" may mean that in a case where the physiological activity exhibited by the biofunctional substance is evaluated using a quantitative index,

the physiological activity (the physiological activity may be, for example, an amount indicating the physiological activity) exhibited by a biofunctional substance when a liquid pharmaceutical composition containing the biofunctional substance and having an osmotic pressure within a predetermined range described below is injected into an injection target using a needle-equipped injector is surpassed by

the physiological activity (the physiological activity may be, for example, an amount indicating the physiological activity) exhibited by a biofunctional substance when a liquid pharmaceutical composition containing the biofunctional substance and having an osmotic pressure within the predetermined range described below is injected into an injection target in the same manner as described above except for using an injector configured to inject the liquid pharmaceutical composition into the injection target without using an injection needle.

That is, for example,

in a case where the physiological activity exhibited by a biofunctional substance when a liquid pharmaceutical composition containing the biofunctional substance and having an osmotic pressure of more than 400 mOsmol/kg and 1000 mOsmol/kg or less is injected into an injection target using a needle-equipped injector (this is referred to as embodiment C) is defined as "activity C", and

the physiological activity exhibited by a biofunctional substance under the same conditions as in the embodiment C except that a liquid pharmaceutical composition containing the biofunctional substance and having an osmotic pressure of more than 400 mOsmol/kg and 1000 mOsmol/kg or less is injected into an injection target using an injector configured to inject the liquid pharmaceutical composition into the injection target without using an injection needle (this is referred to as embodiment A) is defined as "activity A",

$$\text{activity C} < \text{activity A}$$

may be satisfied.

In a case where the physiological activity exhibited by the biofunctional substance is evaluated by a quantitative index (for example, a level of expression, an antibody titer, an efficacy score against a disease, or the like), the quantitative index only needs to be appropriately set depending on the physiological activity exhibited by the biofunctional substance.

In a case where the biofunctional substance is DNA and contains a gene, examples of the quantitative index include an amount of mRNA produced using the DNA as a template and an amount of protein produced using the mRNA as a template (usually evaluated as an expression level of the gene).

In addition, for example, when the biofunctional substance is mRNA, examples of the quantitative index include the amount of protein produced using the mRNA as a template.

That is, for example, in a case where the biofunctional substance is DNA and contains a gene, and the quantitative index is an expression level of the gene,

an expression level of the gene when a liquid pharmaceutical composition having an osmotic pressure of 400 mOsmol/kg or less or more than 1000 mOsmol/kg is injected into an injection target with an injector configured to inject the liquid pharmaceutical composition into the injection target without using an injection needle (this is referred to as embodiment B1) is defined as "expression level B1", and

an expression level of the gene when a liquid pharmaceutical composition is injected into an injection target in the same manner as described above under the same conditions as in the embodiment B1 except that the osmotic pressure is more than 400 mOsmol/kg and 1000 mOsmol/kg or less (this is referred to as embodiment A1) is defined as "expression level A1",

$$\text{expression level B1} < \text{expression level A1}$$

is satisfied.

In a case where an expression level of the gene when a liquid pharmaceutical composition having an osmotic pressure of more than 400 mOsmol/kg and 1000 mOsmol/kg or less is injected into an injection target using a needle-equipped injector (this is referred to as embodiment C1) is defined as "expression level C1", and
an expression level of the gene under the same conditions as in the embodiment C1 except that a liquid pharmaceutical composition having an osmotic pressure of more than 400 mOsmol/kg and 1000 mOsmol/kg or less is injected into an injection target with an injector configured to inject the liquid pharmaceutical composition into the injection target without using an injection needle (this is referred to as embodiment A1) is defined as "expression level A1",

$$\text{expression level C1} < \text{expression level A1}$$

may be satisfied.

When the biofunctional substance is a peptide or a protein, examples of the quantitative index are as follows.

For example, when the injection target is an injection target capable of producing an antibody (for example, an individual (living body)) and the peptide or the protein causes production of an antibody against the peptide or the protein in the injection target (antigen or the like), examples of the quantitative index include the antibody titer of the antibody.

For example, when the injection target is an injection target capable of inducing cellular immunity (for example, an individual (living body)) and the peptide or the protein induces cellular immunity in the injection target, examples of the quantitative index include the amount of cytokine produced, and the number of cytokine-producing cells measured by ELISpot.

For example, when the injection target is an injection target capable of activating immune cells (for example, an individual (living body)) and the peptide or the protein activates immune cells in the injection target, examples of the quantitative index include the amount of CD80 produced, the amount of CD86 produced, the amount of CD40 produced, the amount of MHC molecules produced, and the total number of immune cells.

For example, when the injection target is a disease model (for example, an individual (living body)) and the peptide or the protein ameliorates the disease in the injection target, examples of the quantitative index include a value of the efficacy score against the disease.

The method for evaluating the quantitative index can be appropriately selected, for example, depending on the physiological activity exhibited by the biofunctional substance using the entirety or a part of an injection target after the liquid pharmaceutical composition is injected into the injection target.

For example, in a case where the injection target is an individual (living body), the biofunctional substance is DNA containing a gene, and the expression level of the gene in the vicinity of an injection site (i.e., a part of the injection target) is evaluated as in Examples described below, after the liquid pharmaceutical composition is injected into the injection target, the vicinity of the injection site is obtained, and the method can be appropriately selected depending on the physiological activity exhibited by the biofunctional substance.

Alternatively, as in Examples described below, the physiological activity exhibited by the biofunctional substance can be evaluated by preparing a sample and measuring an amount of protein produced by a known method. In Examples described below, the protein produced is GFP protein, and thus the physiological activity is evaluated using fluorescent brightness as an index.

For example, in a case where the injection target is an individual (living body), the biofunctional substance is a nucleic acid encoding a protein that induces cellular immunity, and the amount of cellular immunity induced is evaluated, after the liquid pharmaceutical composition is injected into the injection target, the amount of cellular immunity induced can be evaluated using, for example, a known method for evaluating the amount of cellular immunity induced using the individual (living body) itself (i.e., the entire injection target).

(Osmotic Pressure)

When the osmotic pressure of the liquid pharmaceutical composition according to the present embodiment is within a predetermined range, higher physiological activity is achieved when the liquid pharmaceutical composition is injected into an injection target using an injector configured to inject the liquid pharmaceutical composition into the injection target without using an injection needle, as compared with a case where a liquid pharmaceutical composition in the related art is injected.

The lower limit of the predetermined range is, for example, more than 400 mOsmol/kg, 436 mOsmol/kg or more, 460 mOsmol/kg or more, 584 mOsmol/kg or more, 605 mOsmol/kg or more, 624 mOsmol/kg or more, 872 mOsmol/kg or more, or 879 mOsmol/kg or more.

In a case where the injection target is a cell or contains a cell, it is presumed that, when the osmotic pressure is higher, physical stress is likely to occur in the cell membrane due to the difference between the osmotic pressure of the liquid pharmaceutical composition and the osmotic pressure in the cell, the substance permeability of the cell membrane is more likely to be improved, as a result, the amount of the biofunctional substance injected into the cell is more likely to increase, and high physiological activity is more likely to be achieved. Conversely, it is presumed that, when the osmotic pressure is lower, the difference between the osmotic pressure of the liquid pharmaceutical composition and the osmotic pressure in the cell is less likely to increase (or no difference occurs), the physical stress is less likely to occur in the cell membrane, the substance permeability of the cell membrane is less likely to be affected, as a result, the amount of the biofunctional substance injected into the cell is less likely to increase, and high physiological activity is less likely to be achieved.

In a case where the injection target is, for example, an individual (living body), it is presumed that, when the osmotic pressure is higher, local retention of the liquid pharmaceutical composition injected into the injection target is more likely to be improved, and, as a result, high physiological activity is more likely to be achieved in the injection target. Conversely, it is presumed that, when the osmotic pressure is lower, the local retention of the liquid pharmaceutical composition injected into the injection target is more likely to decrease, the liquid pharmaceutical composition is more likely to be decomposed or dispersed, and, as a result, high physiological activity is less likely to be achieved in the injection target.

The upper limit of the predetermined range is, for example, 1000 mOsmol/kg or less, 899 mOsmol/kg or less, 879 mOsmol/kg or less, 872 mOsmol/kg or less, 624 mOsmol/kg or less, 605 mOsmol/kg or less, 584 mOsmol/kg or less, 460 mOsmol/kg or less, or 436 mOsmol/kg or less.

When the osmotic pressure is lower, a load on the injection target due to the injection of the liquid pharmaceutical composition can be suppressed as much as possible. For example, in a case where the injection target is a cell or contains a cell, damage or death of the cell can be suppressed as much as possible. For example, when the injection target is a mammalian individual (living body), the occurrence of internal bleeding at the injection site due to injection of the liquid pharmaceutical composition can be suppressed as much as possible.

The upper limit and the lower limit of the predetermined range may be a consistent combination selected from the examples of the upper limit and the examples of the lower limit described above in view of the reason for adopting the upper limit and the reason for adopting the lower limit. Specifically, the predetermined range may be as follows.

For example, the predetermined range may be more than 400 mOsmol/kg and 1000 mOsmol/kg or less, more than 400 mOsmol/kg and 899 mOsmol/kg or less, more than 400 mOsmol/kg and 879 mOsmol/kg or less, more than 400 mOsmol/kg and 872 mOsmol/kg or less, more than 400 mOsmol/kg and 624 mOsmol/kg or less, more than 400 mOsmol/kg and 605 mOsmol/kg or less, more than 400 mOsmol/kg and 584 mOsmol/kg or less, more than 400 mOsmol/kg and 460 mOsmol/kg or less, or more than 400 mOsmol/kg and 436 mOsmol/kg or less.

For example, the predetermined range may be 436 mOsmol/kg or more and 1000 mOsmol/kg or less, 436 mOsmol/kg or more and 899 mOsmol/kg or less, 436 mOsmol/kg or more and 879 mOsmol/kg or less, 436 mOsmol/kg or more and 872 mOsmol/kg or less, 436 mOsmol/kg or more and 624 mOsmol/kg or less, 436 mOsmol/kg or more and 605 mOsmol/kg or less, 436 mOsmol/kg or more and 584 mOsmol/kg or less, or 436 mOsmol/kg or more and 460 mOsmol/kg or less.

For example, the predetermined range may be 460 mOsmol/kg or more and 1000 mOsmol/kg or less, 460 mOsmol/kg or more and 899 mOsmol/kg or less, 460 mOsmol/kg or more and 879 mOsmol/kg or less, 460 mOsmol/kg or more and 872 mOsmol/kg or less, 460 mOsmol/kg or more and 624 mOsmol/kg or less, 460 mOsmol/kg or more and 605 mOsmol/kg or less, or 460 mOsmol/kg or more and 584 mOsmol/kg or less.

For example, the predetermined range may be 584 mOsmol/kg or more and 1000 mOsmol/kg or less, 584 mOsmol/kg or more and 899 mOsmol/kg or less, 584 mOsmol/kg or more and 879 mOsmol/kg or less, 584 mOsmol/kg or more and 872 mOsmol/kg or less, 584 mOsmol/kg or more and 624 mOsmol/kg or less, or 584 mOsmol/kg or more and 605 mOsmol/kg or less.

For example, the predetermined range may be 605 mOsmol/kg or more and 1000 mOsmol/kg or less, 605 mOsmol/kg or more and 899 mOsmol/kg or less, 605 mOsmol/kg or more and 879 mOsmol/kg or less, 605 mOsmol/kg or more and 872 mOsmol/kg or less, or 605 mOsmol/kg or more and 624 mOsmol/kg or less.

For example, the predetermined range may be 624 mOsmol/kg or more and 1000 mOsmol/kg or less, 624 mOsmol/kg or more and 899 mOsmol/kg or less, 624 mOsmol/kg or more and 879 mOsmol/kg or less, or 624 mOsmol/kg or more and 872 mOsmol/kg or less.

For example, the predetermined range may be 872 mOsmol/kg or more and 1000 mOsmol/kg or less, 872 mOsmol/kg or more and 899 mOsmol/kg or less, or 872 mOsmol/kg or more and 879 mOsmol/kg or less.

For example, the predetermined range may be 879 mOsmol/kg or more and 1000 mOsmol/kg or less, or 879 mOsmol/kg or more and 899 mOsmol/kg or less.

The osmotic pressure of the liquid pharmaceutical composition can be measured using, for example, an osmometer 3000 basic available from Gonotec.

(Injection Target)

The injection target in the present embodiment may be, for example, one or more selected from the group consisting of a cell, a cell sheet, a cell cluster, a tissue, an organ (skin, a body part, or the like), an organ system, an individual (living body), and the like, and is not limited. Any of an in vitro system, an in vivo system, and an ex vivo system may be adoptable. The cell cluster may be a cell cluster obtained by three-dimensional culture, and the organ (skin, body part, or the like) may be an organoid.

When injection is performed to the injection target, the injection may be performed to a lower layer included in the injection target. That is, for example, when an individual (living body) is an injection target, injection may be performed to a tissue included in the individual (living body), may be performed to a cell included in the individual (living body), or may be performed to both of the tissue and the cell. When a tissue is an injection target, injection may be performed to a cell included in the tissue, may be performed to an intercellular matrix included in the tissue, or may be performed to both of the cell and the intercellular matrix.

When the injection target in the present embodiment is one or more selected from the group consisting of a cell, a cell sheet, a cell cluster, a tissue, an organ (skin, a body part, or the like), and an organ system, the injection target may be one or more selected from the group consisting of a cell, a cell sheet, a cell cluster, a tissue, an organ (skin, a body part, or the like), and an organ system existing in an individual (living body), or one or more selected from the group consisting of a cell, a cell sheet, a cell cluster, a tissue, an organ (skin, a body part, or the like), and an organ system not existing in an individual (living body) (for example, in a state extracted or separated from the individual (living body) or a state produced outside the individual (living body)).

The injection target in the present embodiment may be one or more selected from the group consisting of a cell, a cell sheet, a cell cluster, a tissue, an organ (skin, a body part, or the like), and an organ system derived from a stem cell such as an iPS cell (induced pluripotent stem cell), and may be present in an individual (living body) or not present in an individual (living body) (for example, in a state extracted or separated from the individual (living body), or a state produced outside the individual (living body)). The cell cluster may be a cell cluster obtained by three-dimensional culture, and the organ (skin, body part, or the like) may be an organoid.

The individual (living body) is preferably a mammalian individual (living body). The mammal is not particularly limited, but examples of the mammal include human and mammals other than the human. Examples of the mammal other than the human include mouse, rat, guinea pig, hamster, cow, goat, sheep, pig, monkey, dog, and cat.

Regardless of what the injection target of the present embodiment is among the examples described above, when injection is performed to a cell, the injection may be performed to the cytoplasm of the cell, the injection may be performed to the cell nucleus of the cell, or the injection may be performed to both the cytoplasm and the cell nucleus of the cell.

The injection target of the present embodiment is not particularly limited, but is preferably one or more selected from the group consisting of an intradermal part, a subcutaneous part, and a muscle layer located underneath the skin of a mammalian individual (living body). In this case, a method may be employed in which the liquid pharmaceutical composition is ejected from the injector toward the surface of the skin and is injected into the skin, and the liquid pharmaceutical composition is injected into one or more selected from the group consisting of an intradermal part in the skin, a subcutaneous part, and a muscle layer located underneath the skin.

(Liquid Pharmaceutical Composition)

The liquid pharmaceutical composition according to the present embodiment contains the biofunctional substance, and can be mixed with a non-toxic carrier for a pharmaceutical composition. Examples of the non-toxic carrier for a pharmaceutical composition include those described below in the description of the pharmacologically acceptable agent. As necessary, a commonly used additive, such as a stabilizer, a wetting agent, an emulsifier, a binder, or an isotonizing agent, can be appropriately added.

The content of the biofunctional substance relative to the total amount of the liquid pharmaceutical composition according to the present embodiment can be appropriately determined based on, for example, the type of the biofunctional substance, the injection target, or the physiological activity exhibited by the biofunctional substance in the injection target into which the biofunctional substance has been injected. When the liquid pharmaceutical composition contains a pharmacologically acceptable agent described below, the content of the biofunctional substance can be appropriately determined also in consideration of the content of the pharmacologically acceptable agent.

The amount of the liquid pharmaceutical composition injected into the injection target can be appropriately determined based on, for example, the content and type of the biofunctional substance, the injection target, or the physiological activity exhibited by the biofunctional substance in the injection target into which the biofunctional substance has been injected. When the liquid pharmaceutical composition contains a pharmacologically acceptable agent described below, the amount of the liquid pharmaceutical composition injected can be appropriately determined also in consideration of the amount of the pharmacologically acceptable agent injected.

The pH of the liquid pharmaceutical composition according to the present embodiment is not particularly limited as long as, when the liquid pharmaceutical composition is injected into an injection target by an injector configured to inject the liquid pharmaceutical composition into the injection target without using an injection needle, the biofunctional substance exhibits physiological activity in the injection target and is stably present therein, and there is no adverse effect such as destruction of the injection target.

The liquid pharmaceutical composition according to the present embodiment may be in the form of a vaccine.

(Pharmacologically Acceptable Agent)

The liquid pharmaceutical composition according to the present embodiment preferably contains a pharmacologically acceptable agent. The type of the pharmacologically acceptable agent is not particularly limited as long as, when the liquid pharmaceutical composition is injected into an injection target by an injector configured to inject the liquid pharmaceutical composition into the injection target without using an injection needle, the biofunctional substance exhibits physiological activity in the injection target and is stably present therein, and there is no adverse effect such as destruction of the injection target.

The content of the pharmacologically acceptable agent relative to the total amount of the liquid pharmaceutical composition according to the present embodiment can be appropriately determined based on, for example, the content and type of the biofunctional substance, the injection target, or the physiological activity exhibited by the biofunctional substance in the injection target into which the biofunctional substance has been injected.

The pharmacologically acceptable agent may adjust the osmotic pressure of the liquid pharmaceutical composition. When the pharmacologically acceptable agent is a solution, it may be a solvent for the liquid pharmaceutical composition or a solvent for dissolving the biofunctional substance. Even when the pharmacologically acceptable agent is a solid, it may be dissolved in a pharmacologically acceptable liquid (for example, water (for example, water for injection)) and may be a solvent for the liquid pharmaceutical composition or a solvent for dissolving the biofunctional substance.

In the present disclosure, "dissolution" may be "suspension" or "emulsification", "solution" may be "suspension" or "emulsion", and "solvent" may be "suspending agent" or "emulsifier".

The pharmacologically acceptable agent is preferably an ionic agent or a nonionic agent, from the viewpoint of compatibility with the injection target.

Among them, an ionic agent is preferred from the viewpoint of versatility in commercial use.

From the viewpoint of storage stability such as lyophilization, a nonionic agent is preferred.

Among the ionic agents, any known ionic buffer can be used as the ionic buffer. Examples of usable ionic buffers include buffer solutions containing phosphoric acid (e.g., phosphate buffer solution, phosphate buffered saline (PBS) (which may be PBS (+) or PBS (-)), Dulbecco's phosphate buffered saline (D-PBS), citrate-phosphate buffer solution, and citrate-phosphate buffered saline), citrate buffers, trishydroxymethylaminomethane-HCl buffers (tris-hydrochloric acid buffer), acetate buffers, GOOD buffers (e.g., HEPES-NaOH buffer), amino acid-based buffers (e.g., glycine-hydrochloric acid buffer, glycine-NaOH buffer, glycylglycine-NaOH buffer, and glycylglycine-KOH buffer), and imidazole buffers.

From the viewpoint of actual use in pharmaceuticals, known ionic additives for pharmaceuticals can be used. Examples of usable additives include sodium chloride, sodium ascorbate, magnesium sulfate, sodium oleate, sodium acetyltryptophan, amino acids, calcium citrate, monosodium succinate, sodium tartrate, aluminum hydroxide, phospholipids, sodium thioglycolate, sulfuric acid, sodium sulfate, hydrochloric acid, sodium hydrochloride, sodium hydroxide, pH adjusters (e.g., hydrochloric acid, sulfuric acid, and sodium hydroxide), phosphoric acid, sodium phosphate, and sodium dihydrogen phosphate.

Further, from the viewpoint of versatility, sodium chloride or a buffer solution containing phosphoric acid is preferred.

The nonionic agent may be any known nonionic additive for pharmaceuticals. Examples of usable additives include glucose, mannose, mannitol, sorbitol, trehalose, sucrose, glycerin, polyethylene glycol, cholesterol, tocopherol, cyclodextrin, and hydroxypropyl methylcellulose.

Glucose, mannose, mannitol, sorbitol, trehalose, sucrose, glycerin, or polyethylene glycol is preferred from the viewpoint of actual use in pharmaceuticals.

Further, from the viewpoint of versatility, glucose is preferred.

(Injector)

The injector used in the present embodiment may be an injector capable of injecting the liquid pharmaceutical composition into the injection target without using an injection needle. That is, the liquid pharmaceutical composition may be injected into an injection target by an injector configured to inject a liquid pharmaceutical composition into an injection target without using an injection needle.

In the injector according to the present embodiment, the term "tip end side" means a side on which an ejection port through which the liquid pharmaceutical composition is ejected from the injector is disposed, and the term "base end side" means a side opposite to the tip end side in the injector, and these terms do not limitedly indicate a specific portion or position.

In the injector according to the present embodiment, a drive section applies ejection energy to eject the liquid pharmaceutical composition toward the injection target. The "ejection" by the injector according to the present embodiment is realized by using the ejection energy by the drive section, pressurizing the liquid pharmaceutical composition that is stored in the storage section by the pressurization section, and thus causing the liquid pharmaceutical composition to flow through a flow path of the storage section. The ejection energy may be ejection energy to be used in a typical injector, and for example, combustion energy of an explosive or the like, generation energy of a gas generating agent or the like, electric energy of a piezoelectric element or the like, or mechanical energy of a spring or the like may be used, or energy obtained by appropriately combining these forms of energy may be used.

In a case where the combustion energy of the explosive is used as the ejection energy, it is preferable to use, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of a plurality of these explosives in combination. Such an explosive is characterized in that the combustion product thereof is gas at a high temperature but does not contain a gas component at normal temperature, and hence, the combustion product is condensed immediately after the ignition. Thus, during a pressurization process for ejection of the liquid pharmaceutical composition, the temperature of the combustion product at the time of pressurization can be shifted by the combustion of an ignition agent to around the normal temperature in a short period of time after the pressure applied to the liquid pharmaceutical composition reaches a first peak ejection force.

When the generation energy by the gas generating agent is used as the ejection energy, the gas generating agent may be a single base smokeless explosive (for example, a single base smokeless explosive containing 98 mass% of nitrocellulose, 0.8 mass% of diphenylamine, and 1.2 mass% of potassium sulfate), or any of various gas generating agents used in gas generators for air bags and gas generators for seat belt pretensioners.

In the injector according to the present embodiment, the pressurization section pressurizes the liquid pharmaceutical composition that is stored in the storage section during the operation, which causes the liquid pharmaceutical composition to be ejected from the ejection port toward the injection target.

The pressurization by the pressurization section is not particularly limited as long as a system is not destroyed, for example, like the destruction of the storage section, and pressurization conditions of an ordinary injector may be employed.

In this context, the pressure means a pressure in the storage section. The method for measuring the pressure is not particularly limited, and for example, measurement can be performed as follows. That is, as in the measurement method described in JP 2005-21640 A, measurement is performed by a method in which an ejection force is applied in a dispersed manner to a diaphragm of a load cell disposed downstream of a nozzle and an output from the load cell is collected by a data collection device via a detection amplifier and is stored as an ejection force (N) per unit time. When the ejection pressure measured in this manner is divided by the area of an ejection port 31a of the injector, the ejection pressure is calculated. The measurement value obtained by the internal pressure measurement of the storage section is equivalent to the ejection pressure, and the ejection pressure can be regarded as the pressure inside the storage section.

The ejection energy by the drive section is transmitted to a plunger through a piston, and the plunger slides in the storage section, whereby the liquid pharmaceutical composition that is stored in the storage section is pushed out along the flow path formed in the nozzle section, and finally ejected from the ejection port toward the injection target.

The liquid pharmaceutical composition may or may not be stored in the storage section from the beginning. In a case where the liquid pharmaceutical composition is not stored in the storage section, the liquid pharmaceutical composition can be stored in the storage section by being aspirated into the storage section through a nozzle including an ejection port. Employing the configuration that requires the storing operation into the storage section in this manner allows any necessary liquid pharmaceutical composition to be injected into the injection target. For this reason, in the injector according to the present embodiment, a syringe section and an injector body may be detachably configured.

In the present embodiment, the injection of the liquid pharmaceutical composition into the injection target by the injector configured to inject a liquid pharmaceutical composition into an injection target without using an injection needle may be injection into the injection target by jet injection.

The term "jet injection" in the present disclosure refers to injection without using an injection needle, characterized in that a liquid pharmaceutical composition containing a biofunctional substance and having an osmotic pressure of more than 400 mOsmol/kg and 1000 mOsmol/kg or less is ejected from an ejection port toward an injection target to form a through hole penetrating the boundary between the inside and the outside of the injection target, and a highpressure ultrafine liquid flow capable of injecting the liquid pharmaceutical composition into the injection target is generated through the through hole. For example, in a case where the injection target is a mammalian individual (living body), the jet injection refers to injection characterized by generating a highpressure ultrafine liquid flow capable of penetrating the skin of the mammalian individual (living body), for example.

The injector according to the present embodiment can be obtained by storing the liquid pharmaceutical composition in the storage section in a typical injector that can be used to inject an injection solution (such as the liquid pharmaceutical composition) into an injection target without using an injection needle. Examples of such a typical injector include the injector described in WO 2019/156238, the injector described in WO 2019/156239, the injector described in WO 2019/156237, and the injector described in JP 5989039 B. These are injectors capable of jet injection, but there are many other commercially available injectors (needleless injectors) capable of the jet injection. Examples thereof include Straits (Pharmajet), Tropis (Pharmajet), Vitajet (Bioject Medical Technologies Inc.), Biojector 2000 (Bioject Medical Technologies Inc.), Bioject Zetajet (Bioject Medical Technologies Inc.), Glide (Glide Pharma), MediJector Vision (Antares Inc.), Sumaval DosePro (Zogenix Inc.), SQ Pen (Bespak), Injex (Equidyne), and hyaluronic acid syringe (BEAUTTO, Amazon Standard Identification Number (ASIN): B08NCHTRHZ).

With reference to the drawings, an injector 1 (needleless injector) will be described below as an example of the injector according to the present embodiment. Note that a configuration according to the following embodiment is provided as an example, and the disclosure is not limited to the configuration according to the present embodiment. The terms "tip end side" and "base end side" are used as terms indicating a relative positional relationship in a longitudinal direction of the injector 1. The term "tip end side" indicates a side close to the tip end of the injector 1 to be described below, that is, a position close to an ejection port 31a, and the term "base end side" indicates a direction opposite to the "tip end side" in the longitudinal direction of the injector 1, that is, a direction toward a side of a drive section 7. The present example is an example in which combustion energy of an explosive ignited by an ignition device is used as ejection energy for pressurization, but the present embodiment is not limited to this example.

(Configuration of Injector 1)

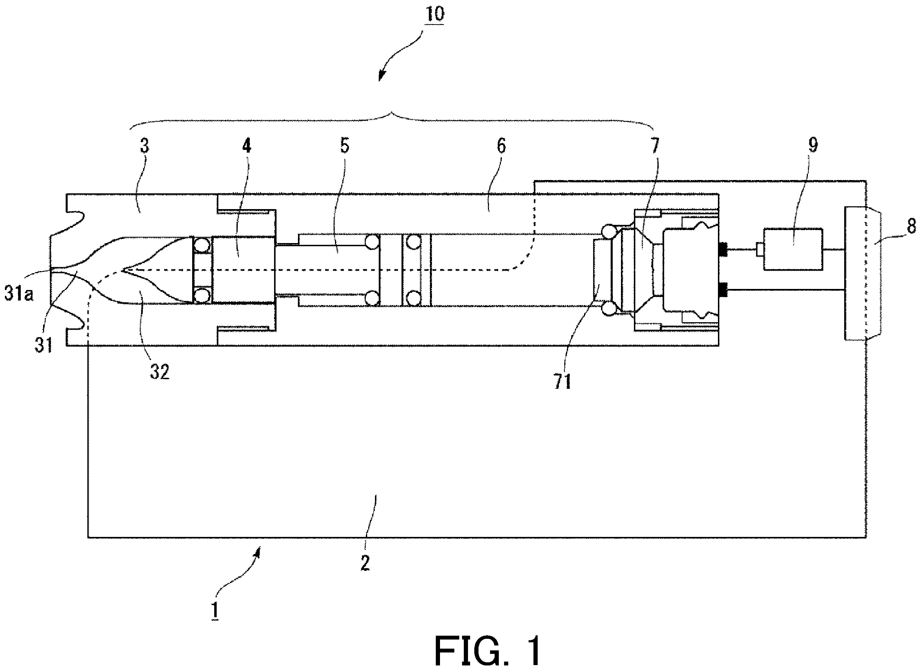

FIG. 1 is a cross-sectional view of the injector 1, taken along the longitudinal direction thereof, illustrating a schematic configuration of the injector 1. The injector 1 is formed by attaching an injector assembly 10 to a housing (injector housing) 2. The injector assembly 10 includes a subassembly including a syringe section 3 and a plunger 4 and a subassembly including an injector body 6, a piston 5, and a drive section 7, and the subassemblies are integrally assembled.

As described above, the injector assembly 10 is configured to be attachable and detachable to and from the housing 2. A storage section 32 formed between the syringe section 3 and the plunger 4 included in the injector assembly 10 is filled with the liquid pharmaceutical composition. The injector assembly 10 is a unit that is disposed after every ejection of the liquid pharmaceutical composition. Meanwhile, a battery 9 that supplies power to an igniter 71 included in the drive section 7 of the injector assembly 10 is included on the housing 2 side. The power supply from the battery 9 is performed between an electrode on the housing 2 side and an electrode on the drive section 7 side of the injector assembly 10 through wiring lines, when a user performs an operation of pressing a button 8 provided on the housing 2. The electrode on the housing 2 side and the electrode on the drive section 7 side of the injector assembly 10 have shapes and positions designed to come into contact with each other automatically when the injector assembly 10 is attached to the housing 2. The housing 2 is a unit that can be repeatedly used as long as there is power remaining in the battery 9 that can be supplied to the drive section 7. Note that when the battery 9 runs out of power in the housing 2, the housing 2 may be continued to be used with only the battery 9 exchanged.

The details of the injector assembly 10 will next be described. First of all, a description is given on the subassembly including the syringe section 3 and the plunger 4. In the syringe section 3, the storage section 32 is formed as a space in which the liquid pharmaceutical composition can be stored. More specifically, as illustrated in FIG. 1, the plunger 4 is disposed slidably along an inner wall surface extending in the axial direction of the syringe section 3, and the storage section 32 is defined by the inner wall surface of the syringe section 3 and the plunger 4. Additionally, the syringe section 3 includes a nozzle section 31 communicating with the storage section 32, and the nozzle section 31 is provided with the ejection port 31a on the tip end side. The nozzle section 31 is a flow path whose cross-sectional area gradually decreases from the storage section 32 side toward the ejection port 31a side, and the flow path is configured to guide the liquid pharmaceutical composition that is filled in the storage section 32 to the ejection port 31a. In the example illustrated in FIG. 1, the plunger 4 has a shape on the tip end side that substantially matches the shape of the nozzle section 31.

Next will be described the subassembly including the injector body 6, the piston 5, and the drive section 7. The piston 5 is made of metal, for example, and is configured to be pressurized by a combustion product (combustion gas) generated by the igniter 71 of the drive section 7 and to slide in a through hole formed in the injector body 6. The injector body 6 is a substantially cylindrical member, and the piston 5 is contained therein slidably along the inner wall surface extending in the axial direction thereof. The piston 5 may be formed of a resin, and in such a case, a metal may be used in combination for a portion required to have heat resistance and pressure resistance. Additionally, as illustrated in FIG. 1, the piston 5 is integrally connected with the plunger 4.

The drive section 7 will next be described. As illustrated in FIG. 1, the drive section 7 is fixed on the base end side with respect to the through hole in the injector body 6. The drive section 7 includes the igniter 71, which is an electric igniter.

The igniter 71 is disposed to face the interior of the through hole in the injector body 6 and contains an ignition agent therein. As the ignition agent, various types of explosives can be employed as described above. The ignition agent can be contained in an explosive cup formed of an appropriate thin metal, for example.

How the injector 1 having the configuration described above is operated will next be described. As illustrated in FIG. 1, in a state where the injector assembly 10 is attached to the housing 2, the liquid pharmaceutical composition is aspirated from the ejection port 31a of the nozzle section 31. This allows the liquid pharmaceutical composition to be filled in the storage section 32. In this state, for example, with the ejection port 31a of the injector 1 being in contact with the injection target, when a user performs an operation of pressing the button 8 provided on the housing 2, this serves as a trigger and actuation power is supplied from the battery 9 to the igniter 71 of the drive section 7, and thus, the igniter 71 is activated. When the igniter 71 is activated, the ignition agent is ignited and thus combusted, and combustion products (flame, combustion gas, and the like) are generated. As a result, an explosive cup of the igniter 71 is ruptured, for example, and the combustion gas of the ignition agent is released into the through hole in the injector body 6. Thus, the pressure in the through hole of the injector body 6 rapidly increases, and the piston 5 is pressed toward the tip end side of the injector body 6. As a result, the piston 5 slides along the inner wall surface of the through hole in the injector body 6 toward the tip end side. As described above, because the plunger 4 is connected integrally with the piston 5, the plunger 4 also slides along the inner wall surface of the syringe section 3 in conjunction with the piston 5. That is, with the plunger 4 pushed toward the nozzle section 31 located on the tip end side of the syringe section 3, the volume of the storage section 32 storing the liquid pharmaceutical composition is reduced, and the liquid pharmaceutical composition is rapidly pressurized. As a result, the liquid pharmaceutical composition that is filled in the storage section 32 is pushed into the nozzle section 31 and is ejected from the ejection port 31a at a high pressure. Thus, the liquid pharmaceutical composition can be injected into the injection target.

Although an additional explosive component is not particularly provided in the injector body 6 illustrated in FIG. 1, a gas generating agent or the like, which is combusted by a combustion product generated by the explosive combustion at the igniter 71 and generates gas, may be provided in the igniter 71 or the through hole of the injector body 6 to adjust a change in pressure applied to the liquid pharmaceutical composition through the piston 5. The configuration in which the gas generating agent is provided in the igniter 71 is a known technique as disclosed in WO 01-031282, JP 2003-25950 A, and the like. One example of the gas generating agent is a single base smokeless explosive containing 98 mass% of nitrocellulose, 0.8 mass% of diphenylamine, and 1.2 mass% of potassium sulfate. Various types of gas generating agents used in a gas generator for an air bag and a gas generator for a seat belt pretensioner may be used. A combustion completion time period of the gas generating agent can be changed by adjusting the dimensions, size, shape, and particularly, surface shape of the gas generating agent when provided in the through hole, which allows a change in pressure applied to the liquid pharmaceutical composition to be adjusted to a desired change, that is, a change that causes the liquid pharmaceutical composition to appropriately reach the injection target. In the present embodiment, the gas generating agent to be used as necessary is also included in the drive section 7. In the present embodiment, the plunger 4 and the piston 5 constitute the "pressurization section".

Another embodiment of the present disclosure is an injector configured to inject a liquid pharmaceutical composition into an injection target without using an injection needle,
the injector including:

a storage section containing the liquid pharmaceutical composition;
a nozzle section communicating with the storage section, the nozzle section having an ejection port for ejecting the liquid pharmaceutical composition toward the injection target; and
a pressurization section for pressurizing the liquid pharmaceutical composition contained in the storage section during an operation, thereby ejecting the liquid pharmaceutical composition from the ejection port toward the injection target,
wherein the liquid pharmaceutical composition contains a biofunctional substance, and has an osmotic pressure of more than 400 mOsmol/kg and 1000 mOsmol/kg or less.

The injector according to the present embodiment is the injector configured to inject a liquid pharmaceutical composition into an injection target without using an injection needle described in the above embodiment, in which the liquid pharmaceutical composition according to the above embodiment is stored in the storage section. The description of the above embodiment is incorporated in the description of the present embodiment.

Another embodiment of the present disclosure is

a method for injecting a liquid pharmaceutical composition into an injection target, the method including injecting a liquid pharmaceutical composition into an injection target by an injector configured to inject the liquid pharmaceutical composition into the injection target without using an injection needle,
the liquid pharmaceutical composition containing a biofunctional substance and having an osmotic pressure of more than 400 mOsmol/kg and 1000 mOsmol/kg or less.

The method according to the present embodiment is a method for injecting the liquid pharmaceutical composition according to the above embodiment into an injection target using the injector according to the above embodiment configured to inject the liquid pharmaceutical composition into the injection target without using an injection needle. The description of the above embodiment is incorporated in the description of the present embodiment.

When the liquid pharmaceutical composition is injected into an injection target by the method according to the present embodiment, the physiological activity of the biofunctional substance contained in the liquid pharmaceutical composition is exhibited in the injection target as described in the above embodiment.

Thus, the method according to the present embodiment may be a method for allowing a biofunctional substance to exhibit physiological activity in an injection target, the method including

injecting a liquid pharmaceutical composition containing a biofunctional substance and having an osmotic pressure of more than 400 mOsmol/kg and 1000 mOsmol/kg or less into an injection target using an injector configured to inject the liquid pharmaceutical composition into the injection target without using an injection needle.

EXAMPLES

Examples will be described below, but none of the examples should be construed as limiting the present disclosure.

The following experiments on animals were conducted in the Institute of Experimental Animal Sciences, Faculty of Medicine, Osaka University and were carried out in accordance with Osaka University Regulations on Animal Experiments defined by the Animal Experiments Committee, Osaka University.

The osmotic pressures of the liquid pharmaceutical compositions used in the following Test Examples were measured using an osmometer 3000 basic available from Gonotec.

[Test Example 1]

In this test example, plasmid DNA containing a GFP gene was used as a biofunctional substance, and phosphate buffered saline (PBS (-)) was used as a pharmacologically acceptable agent (solvent). The injector used was the injector illustrated in FIG. 1 having a nozzle diameter of 0.1 mm. 25 mg of ZPP was used as an ignition agent, and 40 mg of a single base smokeless explosive (containing 98 mass% of nitrocellulose, 0.8 mass% of diphenylamine, and 1.2 mass% of potassium sulfate) was used as a gas generating agent.

(Preparation of Liquid Pharmaceutical Composition)

PBS-tablet (available from Takara Bio Inc.) (PBS(-)) was dissolved in water for injection (available from Otsuka Pharmaceutical Co., Ltd.) to prepare 10-fold PBS. The prepared 10-fold PBS was diluted with the same water for injection so that the concentration thereof was twice the concentration when the PBS was injected into a 10-week-old male balb/c mouse (CLEA Japan, Inc.) as a test animal. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, the final concentration of the plasmid DNA containing a GFP gene was adjusted to 0.5 mg/mL.

Then, liquid pharmaceutical compositions were prepared so as to have a final osmotic pressure (the final magnification of PBS is shown in parentheses) of 198.00 mOsmol/kg (0.5-fold PBS), 346.00 mOsmol/kg (1-fold PBS), 624.00 mOsmol/kg (2-fold PBS), 899.00 mOsmol/kg (3-fold PBS), 1154.00 mOsmol/kg (4-fold PBS), or 1424.00 mOsmol/kg (5-fold PBS).

(Injection into 10-week-old male balb/c mouse)

A 10-week-old male balb/c mouse was anesthetized by aspiration with isoflurane, and then its back was clipped using animal hair clippers. Thereafter, the back was disinfected with alcohol, and the liquid pharmaceutical composition was injected into a plurality of sites of the back using the above-described injector. 20 μL of the liquid pharmaceutical composition per site was injected.

(Evaluation of Gene Expression)

One day after the injection, the mouse was euthanized. Thereafter, a piece of skin having a thickness sufficient for microscopic observation was collected from the back, attached to a Matsunami glass bottom dish, and then observed with a fluorescent microscope BZ-X710 (available from Keyence Corporation). A bright field image (exposure time: 1/350 s) and a fluorescent image of the GFP channel (exposure time: 1/20 s) were acquired, and as necessary, a superimposed image (bright field image + fluorescent image) was acquired.

(Analysis of Gene Expression)

The fluorescent image was analyzed using an analysis application (Hybrid Cell Count) (available from Keyence Corporation). The analysis was performed under the conditions of a brightness setting of 10 and a region designation of off.

[Comparative Test Example 1]

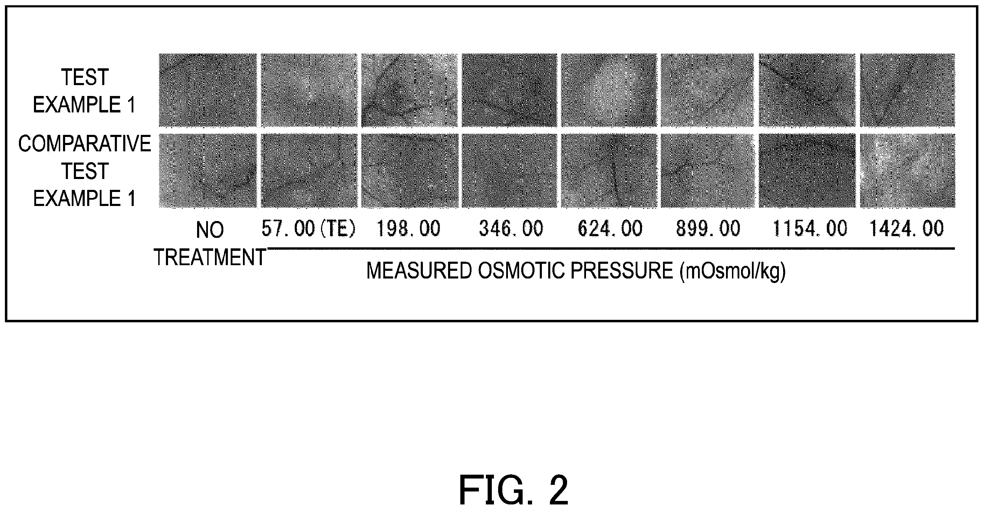

The test was carried out in the same manner as in Test Example 1 except that a needle-equipped injector with a 30G needle (Dendronics needle 30G available from Misawa Medical Industry Co., Ltd.) was used as the injector.
The test was conducted also in a case of no treatment (in a case where the liquid pharmaceutical composition was not injected), in a case where the injector illustrated in FIG. 1 was used as the injector and TE (Tris-EDTA, pH: 8.0) was used as the liquid pharmaceutical composition, and in a case where a needle-equipped injector with a 30G needle (Dendronics needle 30G available from Misawa Medical Industry Co., Ltd.) was used as the injector and TE (Tris-EDTA, pH: 8.0) was used as the liquid pharmaceutical composition.
The results are shown in Table 1 and FIG. 2. "Relative luminous intensity (1)" in the table is a value obtained by performing conversion on the assumption that the luminous intensity in a case of using 57.00 mOsmol/kg (TE) is 1.00, and "Relative luminous intensity (2)" is a value obtained by performing conversion on the assumption that the luminous intensity in a case of using 346.00 mOsmol/kg (1-fold PBS) is 1.00.
Comparison between the conditions of Test Example 1 suggested that, for example, the luminous intensity when the osmotic pressure was more than 400 mOsmol/kg and 1000 mOsmol/kg or less was higher than the luminous intensity when the osmotic pressure was 400 mOsmol/kg.
At any osmotic pressure, the luminous intensity in Test Example 1 was higher than that in Comparative Test Example 1. As shown from the results of the relative luminous intensity (2) (which may be referred to as gene expression increase rate), when the injector illustrated in FIG. 1 was used and the osmotic pressure was 624.00 mOsmol/kg (2-fold PBS) and 899.00 mOsmol/kg (3-fold PBS), the relative luminous intensity was more than 1 time. Also in comparison with the corresponding relative luminous intensity (2) (which may be referred to as gene expression increase rate) of Comparative Test Example 1, the relative luminous intensity was a higher value in Test Example 1. These results revealed that the gene expression enhancing effect can be achieved in association with an increase in osmotic pressure.
Interestingly, it was confirmed that the gene expression increase rate exceeded 1 depending on the osmotic pressure even in Comparative Test Example 1. The results suggest that an increase in osmotic pressure leads to an increase in gene expression even when a needle-equipped injector is used. However, the gene expression increase rate in Test Example 1 was higher than that in Comparative Test Example 1. This revealed that the gene expression enhancing effect associated with an increase in osmotic pressure was particularly remarkable when the injector illustrated in FIG. 1 was used.

[Table 1]

Table 1

| | No treatment | TE | 0.5-Fold PBS | 1-Fold PBS | 2-Fold PBS | 3-Fold PBS | 4-Fold PBS | 5-Fold PBS |
|---|---|---|---|---|---|---|---|---|
| Measured osmotic pressure (mOsmol/kg) | | 57.00 | 198.00 | 346.00 | 624.00 | 899.00 | 1154.00 | 1424.00 |

(continued)

| | | No treatment | TE | 0.5-Fold PBS | 1-Fold PBS | 2-Fold PBS | 3-Fold PBS | 4-Fold PBS | 5-Fold PBS |
|---|---|---|---|---|---|---|---|---|---|
| Test Example 1 | Luminous intensity (a.u.) | 0.00 | 975.94 | 1937.68 | 4603.52 | 25317.49 | 9835.72 | 1835.73 | 713.81 |
| | Relative luminous intensity (1) | | 1.00 | 1.99 | 4.72 | 25.94 | 10.08 | 1.88 | 0.73 |
| | Relative luminous intensity (2) | | | | 1.00 | 5.50 | 2.14 | 0.40 | 0.16 |
| Comparative Test Example 1 | Luminous intensity (a.u.) | 0.00 | 24.71 | 43.04 | 34.56 | 35.59 | 24.95 | 11.52 | 10.91 |
| | Relative luminous intensity (1) | | 1.00 | 1.74 | 1.40 | 1.44 | 1.01 | 0.47 | 0.44 |
| | Relative luminous intensity (2) | | | | 1.00 | 1.03 | 0.72 | 0.33 | 0.32 |

[Test Example 2]

This test example was carried out in the same manner as in Test Example 1 except that sodium chloride (NaCl) dissolved in water for injection (available from Otsuka Pharmaceutical Co., Ltd.) was used as a pharmacologically acceptable agent (solvent), and a 11-week-old male balb/c mouse (CLEA Japan, Inc.) was used as a test animal.

[Comparative Test Example 2]

The test was carried out in the same manner as in Test Example 2 except that a needle-equipped injector with a 30G needle (Dendronics needle 30G available from Misawa Medical Industry Co., Ltd.) was used as the injector.

The test was conducted also in a case where the injector illustrated in FIG. 1 was used as the injector and TE (Tris-EDTA, pH: 8.0) was used as the liquid pharmaceutical composition, and in a case where a needle-equipped injector with a 30G needle (Dendronics needle 30G available from Misawa Medical Industry Co., Ltd.) was used as the injector and TE (Tris-EDTA, pH: 8.0) was used as the liquid pharmaceutical composition.

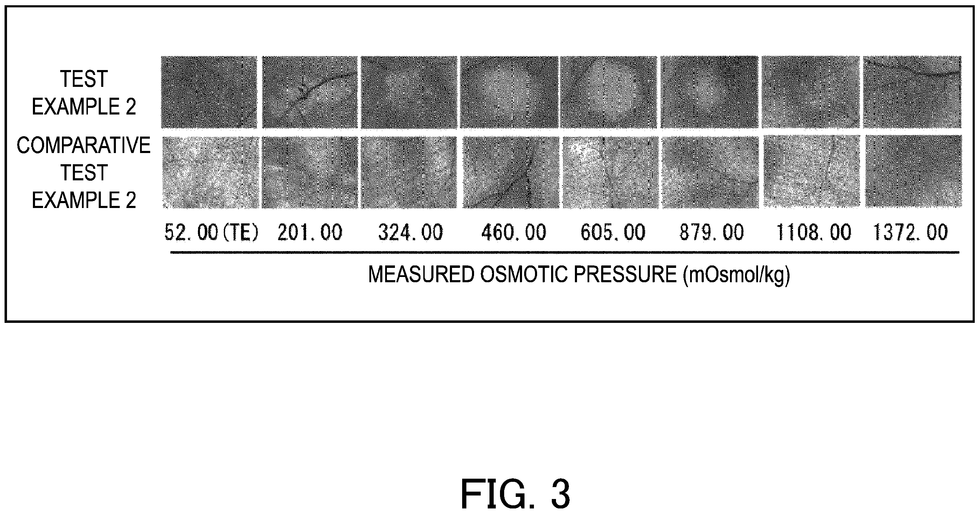

The results are shown in Table 2 and FIG. 3. The concentration in the uppermost row in the table is the concentration of NaCl in the liquid pharmaceutical composition, and "Relative luminous intensity (1)" is a value obtained by performing conversion on the assumption that the luminous intensity in a case of using 52.00 mOsmol/kg (TE) is 1.00, and "Relative luminous intensity (2)" is a value obtained by performing conversion on the assumption that the luminous intensity in a case of using 324.00 mOsmol/kg (150 mM) is 1.00.

Comparison between the conditions of Test Example 2 suggested that, for example, the luminous intensity when the osmotic pressure was more than 400 mOsmol/kg and 1000 mOsmol/kg or less was higher than the luminous intensity when the osmotic pressure was 400 mOsmol/kg.

At any osmotic pressure, the luminous intensity in Test Example 2 was higher than that in Comparative Test Example 2. As shown from the results of the relative luminous intensity (2) (which may be referred to as gene expression increase rate), when the injector illustrated in FIG. 1 was used and the osmotic pressure was 460.00 mOsmol/kg (225 mM), 605.00 mOsmol/kg (300 mM), and 879.00 mOsmol/kg (450 mM), the relative luminous intensity was more than 1 time. Therefore, it was revealed that the gene expression enhancing effect associated with an increase in osmotic pressure can be achieved regardless of the type of salt used.

[Table 2]

Table 2

|  |  | TE | 75 mM | 150 mM | 225 mM | 300 mM | 450 mM | 600 mM | 750 mM |
|---|---|---|---|---|---|---|---|---|---|
| Measured osmotic pressure (mOsmol/kg) | | 52.00 | 201.00 | 324.00 | 460.00 | 605.00 | 879.00 | 1108.00 | 1372.00 |
| Test Example 2 | Luminous intensity (a.u.) | 2413.64 | 6633.35 | 5820.90 | 31693.40 | 34139.70 | 9304.31 | 5187.74 | 1014.51 |
|  | Relative luminous intensity (1) | 1.00 | 2.75 | 2.41 | 13.13 | 14.14 | 3.85 | 2.15 | 0.42 |
|  | Relative luminous intensity (2) |  |  | 1.00 | 5.44 | 5.87 | 1.60 | 0.89 | 0.17 |
| Comparative Test Example 2 | Luminous intensity (a.u.) | 84.64 | 15.41 | 96.04 | 110.58 | 2.56 | 33.75 | 66.51 | 115.03 |
|  | Relative luminous intensity (1) | 1.00 | 0.18 | 1.13 | 1.31 | 0.03 | 0.40 | 0.79 | 1.36 |
|  | Relative luminous intensity (2) |  |  | 1.00 | 1.15 | 0.03 | 0.35 | 0.69 | 1.20 |

[Test Example 3]

This test example was carried out in the same manner as in Test Example 1 except that glucose dissolved in water for injection (available from Otsuka Pharmaceutical Co., Ltd.) was used as a pharmacologically acceptable agent (solvent), and a 11-week-old male balb/c mouse (CLEA Japan, Inc.) was used as a test animal.

[Comparative Test Example 3]

The test was carried out in the same manner as in Test Example 3 except that a needle-equipped injector with a 30G needle (Dendronics needle 30G available from Misawa Medical Industry Co., Ltd.) was used as the injector.

The test was conducted also in a case where the injector illustrated in FIG. 1 was used as the injector and TE (Tris-EDTA, pH: 8.0) was used as the liquid pharmaceutical composition, and in a case where a needle-equipped injector with a 30G needle (Dendronics needle 30G available from Misawa Medical Industry Co., Ltd.) was used as the injector and TE (Tris-EDTA, pH: 8.0) was used as the liquid pharmaceutical composition.

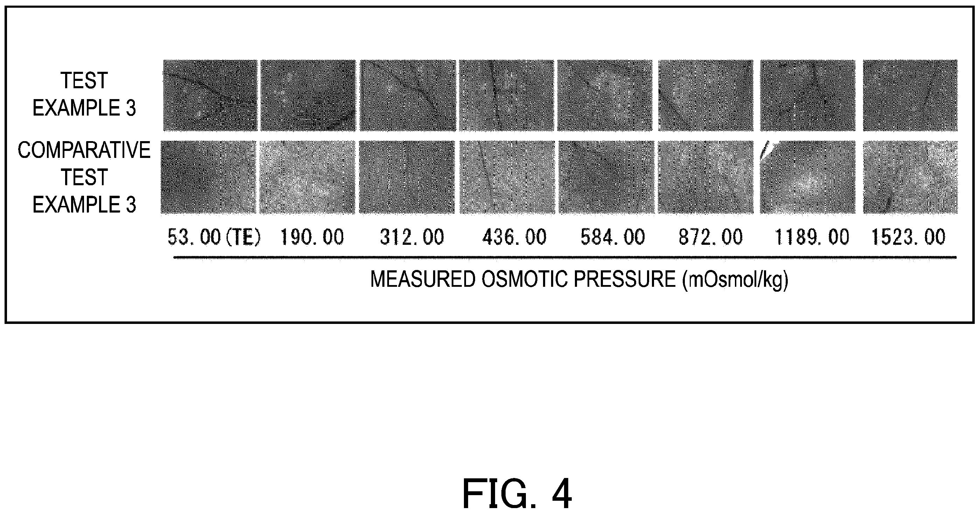

The results are shown in Table 3 and FIG. 4. The concentration in the uppermost row in the table is the concentration of glucose in the liquid pharmaceutical composition, and "Relative luminous intensity (1)" is a value obtained by performing conversion on the assumption that the luminous intensity in a case of using 53.00 mOsmol/kg (TE) is 1.00, and "Relative luminous intensity (2)" is a value obtained by performing conversion on the assumption that the luminous intensity in a case of using 312.00 mOsmol/kg (54 mg/mL) is 1.00.

Comparison between the conditions of Test Example 3 suggested that, for example, the luminous intensity when the osmotic pressure was more than 400 mOsmol/kg and 1000 mOsmol/kg or less was higher than the luminous intensity when the osmotic pressure was 400 mOsmol/kg.

At any osmotic pressure, the luminous intensity in Test Example 3 was higher than that in Comparative Test Example 3. As shown from the results of the relative luminous intensity (2) (which may be referred to as gene expression increase rate), when the injector illustrated in FIG. 1 was used and the osmotic pressure was 436.00 mOsmol/kg (81 mg/mL), 584.00 mOsmol/kg (108 mg/mL), and 872.00 mOsmol/kg (162 mg/mL), the relative luminous intensity was more than 1 time. Therefore, it was shown that the gene expression enhancing effect associated with an increase in osmotic pressure can be achieved regardless of the type of solute used.

[Table 3]

Table 3

| | | TE | 27 mg.ml | 54 mg/mL | 81 mg/mL | 108 mg.mL | 162 mg/mL | 216 mg/mL | 270 mg/mL |
|---|---|---|---|---|---|---|---|---|---|
| Measured osmotic pressure (mOsmol/kg) | | 53.00 | 190.00 | 312.00 | 436.00 | 584.00 | 872.00 | 1189.00 | 1523.00 |
| Test Example 3 | Luminous intensity (a.u.) | 714.05 | 1195.96 | 1003.61 | 2284.72 | 2980.49 | 4487.94 | 271.18 | 481.19 |
| | Relative luminous intensity (1) | 1.00 | 1.67 | 1.41 | 3.20 | 4.17 | 6.29 | 0.38 | 0.67 |
| | Relative luminous intensity (2) | | | 1.00 | 2.28 | 2.97 | 4.47 | 0.27 | 0.48 |
| Comparative Test Example 3 | Luminous intensity (a.u.) | 8.87 | 12.76 | 14.26 | 18.98 | 33.34 | 0.53 | 7.89 | 7.41 |
| | Relative luminous intensity (1) | 1.00 | 1.44 | 1.61 | 2.14 | 3.76 | 0.06 | 0.89 | 0.84 |
| | Relative luminous intensity (2) | | | 1.00 | 1.33 | 2.34 | 0.04 | 0.55 | 0.52 |

[Test Example 4]

This test example was carried out in the same manner as in Test Example 1 except that a spring-type needleless injector (BEAUTTO, hyaluronic acid syringe, Amazon Standard Identification Number (ASIN): B08NCHTRHZ) was used as the injector, and a 11-week-old male balb/c mouse (CLEA Japan Inc.) was used as a test animal.

[Comparative Test Example 4]

The test was carried out in the same manner as in Test Example 4 except that a needle-equipped injector with a 30G needle (Dendronics needle 30G available from Misawa Medical Industry Co., Ltd.) was used as the injector.
The test was conducted also in a case of no treatment (in a case where the liquid pharmaceutical composition was not injected), in a case where a spring-type needleless injector (BEAUTTO, hyaluronic acid syringe, Amazon Standard Identification Number (ASIN): B08NCHTRHZ) was used as the injector and TE (Tris-EDTA, pH: 8.0) was used as the liquid pharmaceutical composition, and in a case where a needle-equipped injector with a 30G needle (Dendronics needle 30G available from Misawa Medical Industry Co., Ltd.) was used as the injector and TE (Tris-EDTA, pH: 8.0) was used as the liquid pharmaceutical composition.

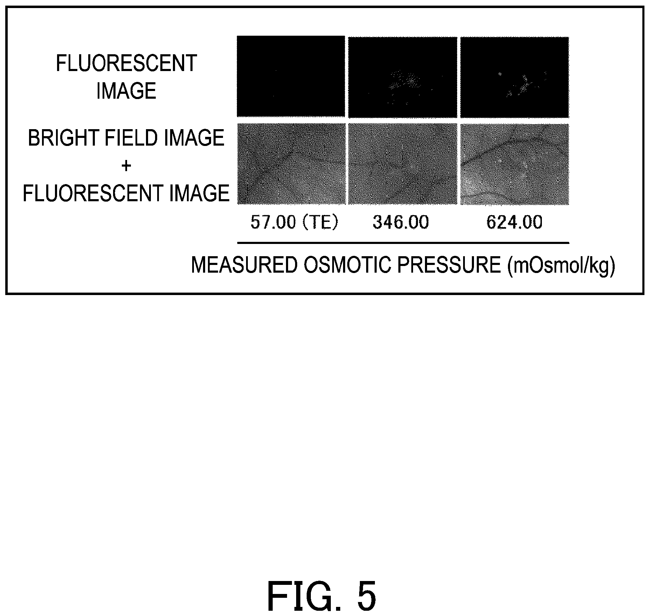

The results are shown in Table 4 and FIG. 5. "Relative luminous intensity (1)" in the table is a value obtained by performing conversion on the assumption that the luminous intensity in a case of using 57.00 mOsmol/kg (TE) is 1.00, and "Relative luminous intensity (2)" is a value obtained by performing conversion on the assumption that the luminous intensity in a case of using 346.00 mOsmol/kg (1-fold PBS) is 1.00.

Comparison between the conditions of Test Example 4 suggested a high probability of showing the same tendency as that of Test Example 1.

The optimum conditions as ejection conditions of the spring-type needleless injector were not examined, and thus gene expression remained low in all cases. However, as shown from the results of the relative luminous intensity (2) (which may be referred to as gene expression increase rate), when the osmotic pressure was 624.00 mOsmol/kg (2-fold PBS), the relative luminous intensity was more than 1 time. Accordingly, it was shown that the gene expression enhancing effect associated with an increase in osmotic pressure is not limited to the injector using the combustion energy of the ignition agent as the ejection energy, but can also be obtained by the spring-type needleless injector.

[Table 4]

Table 4

|  |  | No treatment | TE | 1-Fold PBS | 2-Fold PBS |
|---|---|---|---|---|---|
| Measured osmotic pressure (mOsmol/kg) | | | 57.00 | 346.00 | 624.00 |
| Test Example 4 | Luminous intensity (a.u.) | 0.00 | 7.95 | 228.70 | 726.00 |
| | Relative luminous intensity (1) | | 1.00 | 28.76 | 91.30 |
| | Relative luminous intensity (2) | | | 1.00 | 3.17 |
| Comparative Test Example 4 | Luminous intensity (a.u.) | 0.00 | 24.71 | 34.56 | 35.59 |
| | Relative luminous intensity (1) | | 1.00 | 1.40 | 1.44 |
| | Relative luminous intensity (2) | | | 1.00 | 1.03 |

[Test Example 5]

This test example was carried out in the same manner as in Test Example 1 except that mRNA (unmodified) encoding GFP as a reporter protein (available from OZ, Funakoshi Cat# MRNA15-100) (solvent: TE) (0.2 mg/mL) was used as a biofunctional substance, a 11-week-old male balb/c mouse (CLEA Japan, Inc.) was used as a test animal, and the final mRNA concentration was 0.1 mg/mL (2.0 μg per injection).

[Comparative Test Example 5]

The test was carried out in the same manner as in Test Example 5 except that a needle-equipped injector with a 30G needle (Dendronics needle 30G available from Misawa Medical Industry Co., Ltd.) was used as the injector.

The test was conducted also in a case of no treatment (in a case where the liquid pharmaceutical composition was not injected), in a case where the injector illustrated in FIG. 1 was used as the injector and TE (Tris-EDTA, pH: 8.0) was used as the liquid pharmaceutical composition, and in a case where a needle-equipped injector with a 30G needle (Dendronics needle 30G available from Misawa Medical Industry Co., Ltd.) was used as the injector and TE (Tris-EDTA, pH: 8.0) was used as the liquid pharmaceutical composition.

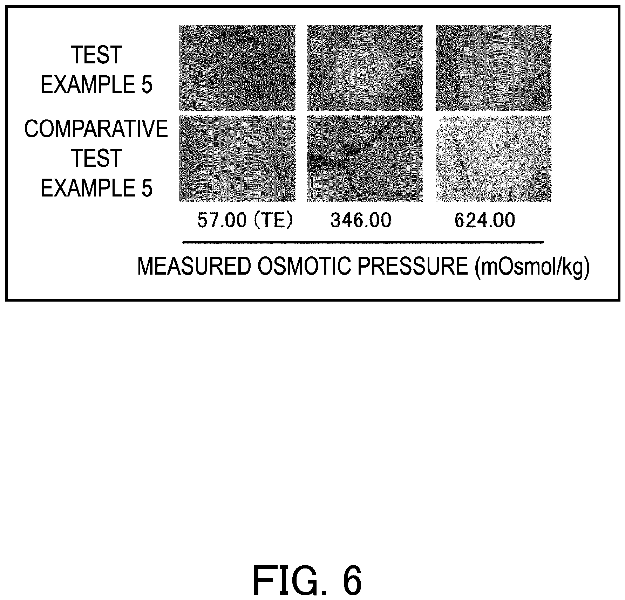

The results are shown in Table 5 and FIG. 6. "Relative luminous intensity (1)" in the table is a value obtained by performing conversion on the assumption that the luminous intensity in a case of using 57.00 mOsmol/kg (TE) is 1.00, and "Relative luminous intensity (2)" is a value obtained by performing conversion on the assumption that the luminous intensity in a case of using 346.00 mOsmol/kg (1-fold PBS) is 1.00.

Comparison between the conditions of Test Example 5 suggested a high probability of showing the same tendency as that of Test Example 1.

As shown from the results of the relative luminous intensity (2) (which may be referred to as gene expression increase rate), when the injector illustrated in FIG. 1 was used and the osmotic pressure was 624.00 mOsmol/kg (2-fold PBS), the relative luminous intensity was more than 1 time. Therefore, it was shown that the gene expression enhancing effect associated with an increase in osmotic pressure can be achieved by a nucleic acid in a broad sense including mRNA.

[Table 5]

Table 5

|  |  | No treatment | TE | 1-Fold PBS | 2-Fold PBS |
|---|---|---|---|---|---|
| Measured osmotic pressure (mOsmol/kg) |  |  | 57.00 | 346.00 | 624.00 |
| Test Example 5 | Luminous intensity (a.u.) | 0.00 | 370.80 | 15739.75 | 50569.34 |
|  | Relative luminous intensity (1) |  | 1.00 | 42.45 | 136.38 |
|  | Relative luminous intensity (2) |  |  | 1.00 | 3.21 |
| Comparative Test Example 5 | Luminous intensity (a.u.) | 0.00 | 17006.00 | 16303.00 | 5579.00 |
|  | Relative luminous intensity (1) |  | 1.00 | 0.96 | 0.33 |
|  | Relative luminous intensity (2) |  |  | 1.00 | 0.34 |

REFERENCE SIGNS LIST

1. Injector, 2. Housing, 3. Syringe section, 4. Plunger, 5. Piston, 6. Injector body, 7. Drive section, 8. Button, 9. Battery, 10. Injector assembly, 31. Nozzle section, 31a. Ejection port, 32. Storage section, 71. Igniter

**Claims**

1. A liquid pharmaceutical composition comprising:

   a biofunctional substance wherein,
   the liquid pharmaceutical composition has an osmotic pressure of more than 400 mOsmol/kg and 1000 mOsmol/kg or less, and
   the liquid pharmaceutical composition is injected into an injection target by an injector configured to inject the liquid pharmaceutical composition into the injection target without using an injection needle.

2. The liquid pharmaceutical composition according to claim 1, comprising a pharmacologically acceptable agent.

3. The liquid pharmaceutical composition according to claim 2, wherein the pharmacologically acceptable agent is an ionic agent.

4. The liquid pharmaceutical composition according to claim 3, wherein the ionic agent is a buffer solution containing phosphoric acid.

5. The liquid pharmaceutical composition according to claim 4, wherein the buffer solution containing phosphoric acid is phosphate buffered saline.

6. The liquid pharmaceutical composition according to claim 3, wherein the ionic agent is sodium chloride.

7. The liquid pharmaceutical composition according to claim 2, wherein the pharmacologically acceptable agent is a nonionic agent.

8. The liquid pharmaceutical composition according to claim 7, wherein the nonionic agent is glucose.

9. The liquid pharmaceutical composition according to claim 1 or 2, wherein injection into the injection target using the injector configured to inject the liquid pharmaceutical composition into the injection target without using an injection needle is injection into the injection target by jet injection.

10. The liquid pharmaceutical composition according to claim 1 or 2, wherein the biofunctional substance is a nucleic acid.

11. An injector configured to inject a liquid pharmaceutical composition into an injection target without using an injection needle, the injector comprising:

a storage section containing the liquid pharmaceutical composition;
a nozzle section communicating with the storage section, the nozzle section having an ejection port for ejecting the liquid pharmaceutical composition toward the injection target; and
a pressurization section for pressurizing the liquid pharmaceutical composition contained in the storage section during an operation, thereby ejecting the liquid pharmaceutical composition from the ejection port toward the injection target, wherein
the liquid pharmaceutical composition contains a biofunctional substance, and has an osmotic pressure of more than 400 mOsmol/kg and 1000 mOsmol/kg or less.

12. A method for injecting a liquid pharmaceutical composition into an injection target, the method comprising injecting a liquid pharmaceutical composition into an injection target by an injector configured to inject the liquid pharmaceutical composition into the injection target without using an injection needle, the liquid pharmaceutical composition containing a biofunctional substance and having an osmotic pressure of more than 400 mOsmol/kg and 1000 mOsmol/kg or less.

FIG. 1

|                                | NO<br>TREATMENT | 57.00 (TE) | 198.00 | 346.00 | 624.00 | 899.00 | 1154.00 | 1424.00 |
|--------------------------------|------------------|------------|--------|--------|--------|--------|---------|---------|
| TEST<br>EXAMPLE 1              |                  |            |        |        |        |        |         |         |
| COMPARATIVE<br>TEST<br>EXAMPLE 1 |                |            |        |        |        |        |         |         |

MEASURED OSMOTIC PRESSURE (mOsmol/kg)

FIG. 2

EP 4 527 374 A1

FIG. 3

FIG. 4

FIG. 5

TEST EXAMPLE 5

COMPARATIVE TEST EXAMPLE 5

57.00 (TE)    346.00    624.00

MEASURED OSMOTIC PRESSURE (mOsmol/kg)

# FIG. 6

## INTERNATIONAL SEARCH REPORT

International application No.

**PCT/JP2023/018700**

### A. CLASSIFICATION OF SUBJECT MATTER

*A61K 9/08*(2006.01)i; *A61K 31/7088*(2006.01)i; *A61K 45/00*(2006.01)i; *A61K 47/02*(2006.01)i; *A61K 47/26*(2006.01)i; *A61K 48/00*(2006.01)i; *A61M 5/303*(2006.01)i; *A61P 43/00*(2006.01)i

FI: A61K9/08; A61M5/303; A61K47/02; A61K47/26; A61K45/00; A61K31/7088; A61K48/00; A61P43/00

According to International Patent Classification (IPC) or to both national classification and IPC

### B. FIELDS SEARCHED

Minimum documentation searched (classification system followed by classification symbols)

A61K9/08; A61K31/7088; A61K45/00; A61K47/02; A61K47/26; A61K48/00; A61M5/303; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2020-146056 A (KONINKLIJKE NEDERLANDSE AKADEMIE VAN WETENSCHAPPEN) 17 September 2020 (2020-09-17) claims 1,18,21, paragraphs [0052], [0099], [0253], examples | 1-8, 10, 12 |
| Y | claims 1,18,21, paragraphs [0052], [0099], [0253], examples | 9, 11 |
| X | JP 2019-514998 A (UCB BIOPHARMA SRL) 06 June 2019 (2019-06-06) claim 1, paragraphs [0049], [0080], [0106] | 1-8, 12 |
| Y | claim 1, paragraphs [0049], [0080], [0106] | 9, 11 |
| A | claim 1, paragraphs [0049], [0080], [0106] | 10 |
| X | JP 2022-062014 A (UCB BIOPHARMA SPRL) 19 April 2022 (2022-04-19) claim 1, paragraphs [0066], [0090], [0101], [0117], table 13 | 1-8, 12 |
| Y | | 9, 11 |
| A | | 10 |
| Y | WO 2019/156237 A1 (DAICEL CORP.) 15 August 2019 (2019-08-15) claims | 9, 11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 July 2023** | **08 August 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

| International application No. |
| --- |
| **PCT/JP2023/018700** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2005-527538 A (CYDEX INC.) 15 September 2005 (2005-09-15)<br>    entire text | 1-12 |
| A | JP 2019-532927 A (CYDEX PHARMACEUTICALS, INC.) 14 November 2019 (2019-11-14)<br>    claims 39, 49 | 1-12 |

Form PCT/ISA/210 (second sheet) (January 2015)

EP 4 527 374 A1

**INTERNATIONAL SEARCH REPORT**
Information on patent family members

International application No.

**PCT/JP2023/018700**

| Patent document cited in search report | | | Publication date (day/month/year) | Patent family member(s) | | | Publication date (day/month/year) |
|---|---|---|---|---|---|---|---|
| JP | 2020-146056 | A | 17 September 2020 | US | 2016/0273001 | A1 | |
| | | | | claims 1, 18, 21, paragraphs [0055], [0101], [0258], examples | | | |
| | | | | EP | 3039148 | A1 | |
| | | | | CN | 105637093 | A | |
| | | | | KR | 10-2016-0056899 | A | |
| JP | 2019-514998 | A | 06 June 2019 | US | 2020/0048346 | A1 | |
| | | | | claim 1, paragraphs [0104], [0192], [0218] | | | |
| | | | | EP | 3454899 | A1 | |
| | | | | CN | 109069643 | A | |
| | | | | KR | 10-2019-0008320 | A | |
| JP | 2022-062014 | A | 19 April 2022 | US | 2019/0083617 | A1 | |
| | | | | claim 1, paragraphs [0075], [0099], [0110], [0126], table 13 | | | |
| | | | | EP | 3426294 | A1 | |
| | | | | CN | 108883175 | A | |
| WO | 2019/156237 | A1 | 15 August 2019 | US | 2021/0023302 | A1 | |
| | | | | claims | | | |
| | | | | EP | 3750579 | A1 | |
| | | | | CN | 111699011 | A | |
| JP | 2005-527538 | A | 15 September 2005 | US | 2003/0073665 | A1 | |
| | | | | the whole documents | | | |
| | | | | EP | 1485110 | A1 | |
| | | | | KR | 10-2004-0097181 | A | |
| JP | 2019-532927 | A | 14 November 2019 | US | 2019/0255000 | A1 | |
| | | | | claims 39, 49 | | | |
| | | | | EP | 3512511 | A1 | |
| | | | | KR | 10-2019-0065296 | A | |
| | | | | CN | 110167543 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012061269 A
- JP 2005021640 A
- WO 2019156238 A
- WO 2019156239 A
- WO 2019156237 A
- JP 5989039 B
- WO 01031282 A
- JP 2003025950 A

**Non-patent literature cited in the description**

- *Clin. Cosmet. Investig. Dermatol.*, 01 May 2018, vol. 11, 231-238
- *AAPS PharmSciTech.*, 09 December 2019, vol. 21 (1), 19